Europäisches Patentamt

**European Patent Office**

**Office européen des brevets**

(19)

(11) Veröffentlichungsnummer: **0 173 206**
**A1**

(12) **EUROPÄISCHE PATENTANMELDUNG**

(21) Anmeldenummer: **85110356.4**

(22) Anmeldetag: **19.08.85**

(51) Int. Cl.⁴: **C 12 N 11/02,** C 12 N 11/08, C 12 P 35/00 // C12R1:125

(30) Priorität: **31.08.84 DE 3432060**

(43) Veröffentlichungstag der Anmeldung: **05.03.86**
**Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Schutt, Hermann, Dr., Gellertweg 12, D-5600 Wuppertal 1 (DE)**

(54) **Immobilisierte Zellen von Bacillus subtilis, Immobilisierungsverfahren und Verwendung des Präparats zur Abspaltung der 3-Acetoxygruppe aus 7-beta-Acylamido-cephalosporansäuren.**

(57) Die Erfindung betrifft immobilisierte Zellen von Bacillus subtilis, ein Verfahren zur Herstellung und die Verwendung des immobilisierten Präparates zur Herstellung von 7-β-Acylamido-3-hydroxymethyl-ceph-3-em-4-carbonsäuren aus reversibel geschützten 7-β-Acylamido-cephalosporansäuren.

ACTORUM AG

BAYER AKTIENGESELLSCHAFT          5090 Leverkusen, Bayerwerk

Konzernverwaltung RP
Patentabteilung                   Ad/bo/c


Immobilisierte Zellen von Bacillus subtilis, Immobilisierungsverfahren und Verwendung des Präparats zur Abspaltung der 3-Acetoxygruppe aus 7-ß-Acylamido-cephalo-
sporansäuren

---

Die Erfindung betrifft immobilisierte Zellen von Bacillus subtilis, ein Verfahren zur Herstellung und die Verwendung des immobilisierten Präparates zur Herstellung
von 7-ß-Acylamido-3-hydroxymethyl-ceph-3-em-4-carbonsäuren aus reversibel geschützten 7-ß-Acylamido-cephalosporansäuren.

Es ist bekannt, daß die chemische Abspaltung der 3-Acetoxygruppe aus 7-Amino-cephalosporansäuren nicht möglich
ist, daß jedoch das Enzym Acetylesterase, isoliert z.B.
aus Bacillus subtilis-Zellen, sowohl in löslicher als
auch in immobilisierter Form zur Abspaltung eingesetzt
wurde.

Alle bekanntgewordenen Verfahren haben jedoch folgende
Nachteile, die ihren Einsatz im technischen Maßstab wesentlich einschränken.

Le A 23 204 -Ausland

(1) Nur stark angereichertes Enzym Acetylesterase weist eine ausreichende enzymatische Stabilität auf.

(2) Die Aktivität unreiner Enzympräparate wird durch proteolytischen Abbau schnell zerstört.

(3) Lösliche Enzyme können nur einmal verwendet werden und verunreinigen u.U. die Produkte.

(4) In Ultrafiltern "immobilisierte" Enzyme verlieren ihre Aktivität schnell durch Inaktivierung und Adsorption an die Ultrafiltrationsmembranen.

(5) Die als Enzymträger verwendeten Materialien, wie z.B. Bentonit, gestatten lediglich die physikalische Adsorption des Enzyms, was bei dauerndem Einsatz zu einem kontinuierlichen Verlust an Enzymaktivität von Enzymträgern führt.

(6) Die kovalente Bindung des Enzyms an wenig poröse Träger, wie z.B. Backsteinpulver, Zirkondioxidpulver sowie an Glaspartikeln, führt zu Biokatalysatoren mit geringer Enzymaktivität pro Träger.

Es bestand daher der Bedarf nach einem ohne großen Aufwand herstellbaren aktiven und stabilen Biokatalysator zur Herstellung von 7-ß-Acylamido-3-hydroxymethyl-ceph-3-em-4-carbonsäuren, gegebenenfalls geeignet für den technischen Einsatz.

Le A 23 204

Die Erfindung betrifft nun einen Biokatalysator, bestehend aus immobilisierten Bacillus subtilis-Zellen.

Von den bekannten Immobilisierungsverfahren erwies sich das folgende, in US-Patent 4 212 943 für Glukoseisomerase beschriebene, Verfahrensprinzip für die Herstellung eines physikalisch stabilen Biokatalysators unter Erhalt der Enzymaktivität für Bacillus subtilis-Zellen als am günstigsten:

1.  Ausflockung oder Ausfällung der Bakterienzellen durch Zusatz von Quebracho Tannin und/oder Betz 1180 - einem stark kationischen, flüssigen, polymeren Koagulans in Form eines N,N-Dimethyl-1,3-propandiamin/1-Chlor-2,3-epoxypropan-Copolymerisates (US-P 3 915 904 und US-P 3 953 330).

2.  Quervernetzung und Härtung des Bakterienpolymerisates mit Polyethylenimin und Glutardialdehyd.

3.  Trocknung und Mahlen der Bakterienpolymerisate.

Die Erfindung betrifft weiterhin ein Verfahren zur Herstellung von immobilisierten Bacillus subtilis-Zellen, dadurch gekennzeichnet, daß die Bacillus subtilis-Zellsuspensionen (Fermenterbrühe) zur Ausfällung und Fixierung der Zellen mit dem Fällmittel Tannin, dem Polyethylenimin und Betz 1180 sowie dem Quervernetzungsmittel Glutardialdehyd in bestimmten Mengenverhältnissen versetzt, das als grauer schwammiger Niederschlag ausfal-

Le A 23 204

lende Bakterienpolymerisat über Filter von der Lösung abgesaugt, im Umlufttrockenschrank bei 40°C getrocknet und die so erhaltene braune feste Masse in einer Zahnradmühle auf unterschiedliche Korngrößen gemahlen wird.

Überraschenderweise ist es wichtig, wie Analysenergebnisse zeigen, zur Herstellung eines stabilen, enzymatisch aktiven und mehrfach verwendbaren Biokatalysators die angegebenen Mengenbereiche einzuhalten:

Bei niedrigem Volumenanteil einer 4 gew.-%igen Tanninlösung ( $<$ 2 Vol.-%) niedrigem Polyethylenimingehalt ( $<$ 0,5 Vol.-% einer 4 gew.-%igen Lösung) und vor allem niedrigem Volumenanteil an Betz/Glutardialdehyd ( $<$ 5,5 Vol.-% einer Mischung aus 6 gew.-%iger Betz-, 2,2 Vol.-% Glutardialdehyd-Lösung) werden Biokatalysatoren erhalten, deren katalytische Aktivität bereits nach dem zweiten Einsatz durch Enzymaktivierung, Abrieb der Bakterienzellen und Substrat in - sowie durch Produktzerstörung drastisch irreversibel abnahm.

Bei Volumenanteilen von 2 - 8 Vol.-% Tanninlösung (4 Gew.-%), 0,5 - 5 Vol.-% Polyethyleniminlösung (4 Gew.-%) und 2,0 - 5,5 Vol.-% Betz/Glutardialdehyd-Lösung (6 gew.-%ige Betz und 2,2 Vol.-% Glutardialdehyd-Lösung) werden dagegen stabile Biokatalysatoren erhalten.

Entscheidend für die Aktivität und Lebensdauer des Biokatalysators ist ein hoher Anteil von Betz/Glutardialdehyd-Lösung.

Die vorliegende Erfindung betrifft weiterhin die Verwendung des nach dem erfindungsgemäßen Verfahren hergestellten Biokatalysators zur Herstellung reversibel geschützter 7-ß-Acylamido-3-hydroxymethyl-ceph-3-em-4-carbonsäuren, die nach Abspaltung der Schutzgruppe zu neuen halbsynthetischen Cephalosporinen verwendet werden können.

Als Substrat des hergestellten Biokatalysators kommen die aus der Literatur bekannten Verbindungen, wie z.B. Phenylacetamido-3-acetoxymethyl-ceph-3-em-4-carbonsäure, 7-ß-/N̄'-tert.-butoxycarbonyl7-D-(∝)-phenylglycinamido-3-acetoxymethyl-ceph-3-em-4-carbonsäure und andere reversibel geschützte Cephalosporansäuren mit 3-Acetoxy-Gruppe in Frage.

Diese können chargenweise oder kontinuierlich mit dem Biokatalysator zur Reaktion gebracht werden. Als bevorzugter Konzentrationsbereich für die Substrate kann 1 - 20 Gew.-%, als bevorzugter pH-Bereich 6 - 8 und als bevorzugter Temperaturbereich 20 - 40°C genannt werden.

Überraschenderweise stieg die Ausbeute an 7-ß-Acylamido-3-hydroxymethyl-ceph-3-em-4-carbonsäure bei mehrfachem Einsatz des Biokatalysators an, bis sie einen konstanten Wert erreicht hatte. Überraschend war auch der Befund, daß trotz nahezu gleichem Einsatz an Bakteriensuspension des Bacillus subtilis ATCC 6633 (75 - 90 Vol.-%) die nach dem erfindungsgemäßen Verfahren hergestellten Biokatalysatoren bis zu dreifache Aktivitäten der Biokatalysatoren aufwiesen, die mit anderen Mengenverhältnissen der Komponenten hergestellt wurden.

Le A 23 204

Die erfindungsgemäßen Biokatalysatoren müssen sorgfältig mit dem 10 - 20-fachen Volumen an Wasser oder Puffer von einem pH-Wert von 6 - 7 gewaschen werden, um starke Produktverluste zu vermeiden.

Die erfindungsgemäßen Biokatalysatoren sind mindestens 20 mal ohne Aktivitätsverlust zur Herstellung von 7-ß-Acylamidocephalosporansäuren einsetzbar.

Die Bestimmung der enzymatischen Aktivität des Biokatalysators erfolgt durch die Hochdruckflüssigkeitschromatographie (s. Anhang) oder durch Auswaage des Produktes nach dem Abdampfen des sauren Essigester-Extraktes.

Als Nachweis der Struktur der bei der enzymatischen Reaktion erhaltenen 3-Hydroxymethylgruppe wurde diese mit Dichlorphosphorylisocyanat in Tetrahydrofuran nach DOS 2 914 000 carbamoyliert und in ein mit der Literatur identisches Carbamoyl-Derivat überführt.

Die 7-ß-Acylamido-Schutzgruppe kann nach chemischer Derivatisierung der 3-Hydroxymethylgruppe wie im Fall der N'-tert.-Butoxycarbonyl-Gruppe wie literaturbekannt sauer abgespalten werden oder wie im Fall der 7-ß-Phenylacetamido-Schutzgruppe mit immobilisierter Penicillinacylase aus E.coli nach DOS 2 355 078 abgespalten werden.

Le A 23 204

0173206

Anhang

<u>Analytisches HPLC-Verfahren zur quantitativen Bestimmung von 7-ß-Phenylacetamido-3-acetoxymethyl-ceph-3-em-4-carbonsäure und 7-ß-Phenylacetamido-3-hydroxymethyl-ceph-3-em-4-carbonsäure</u>

| | |
|---|---|
| Gerät: | Hewlett-Packard 1084 B |
| Stationäre Phase: | Lichrosorb RP 18- (250 mm x 4 mm, 5 µm, Merck, Darmstadt) |
| Mobile Phase: | 1 Ampulle (pH-4-Puffer, Merck, Darmstadt, Nr. 9884), 360 ml Acetonitril, aufgefüllt mit Wasser auf 2 l |
| Fluß: | 2 ml/min |
| Ofentemperatur: | 45°C |
| Injektionsvolumen: | 10 µl |
| Wellenlänge: | 240 nm |
| Attenuation: | $2^8$ |
| Konzentration von 7-ß-Phenylacetamido-3-acetoxy-cephalosporansäure: | 10 mg/ml |
| Retentionszeit für 7-ß-Phenylacetamido-3-acetoxymethyl-ceph-3-em-4-carbonsäure: | 5,04 min |
| Retentionszeit für 7-ß-Phenylacetamido-3-hydroxymethyl-ceph-3-em-4-carbonsäure: | 2,5 min |

<u>Le A 23 204</u>

__Beispiel 1__    Herstellung von immobilisierten Zellen
von Bacillus subtilis ATCC 6633

Herstellung der Betz-Glutardialdehydlösung:
Lösung 1: 120 g Betzpolymer (Betz 1180, kationisches Polymer,
hergestellt aus äquimolaren Mengen Epichlorhydrin und N,N-
Dimethyl-1,3-propandiamin)/1000 ml ents. Wasser mit 1 N NaOH
eingestellt auf pH 9,0,

Lösung 2: 178 ml 25 %ige Glutardialdehyd-Lösung/1000 ml
ents. Wasser mit 1 N NaOH eingestellt auf pH 9,0.

Die Lösungen 1 und 2 werden vor Gebrauch im Volumenverhältnis 1:1 gemischt, der pH-Wert gegebenenfalls mit 1 N
NaOH auf pH 9,0 korrigiert.

25 l Bakteriensuspension Bacillus subtilis ATCC 6633 werden nacheinander unter Rühren mit 1,25 l 4 gew.-%iger
Tanninlösung, 0,5 l 4 %iger Polyethylenimin (PEI-600 Molekulargewicht ca. 30 000 - 40 000), 50 %ige wäßrige Lösung (Serva, Heidelberg) sowie mit 3 l der Betz-Glutardial-
dehyd-Lösung versetzt. Nach 15-minütigem Rühren der Bakterienfällung wird diese über eine Filternutsche (D = 25 - 30 cm)
belegt mit Filterpapier abgesaugt.

Das feuchte Bakterienpolymerisat wurde auf Blechen bei
40°C im Umlufttrockenschrank bis zur Gewichtskonstanz
getrocknet. Das getrocknete Bakterienpolymerisat wird durch
eine Zahnradmühle gemahlen und anschließend gesiebt.

Es werden 168,82 g Bakterienpolymerisat und nach Siebung
mit einem Siebanteil von    630 u (6,09 g), 630 - 200 u

__Le A 23 204__

(115,99 g) und 200 µ (35,86 g) erhalten. Gesamtausbeute nach Siebung: 157,94 g (93,5 %) mit einer Kompressibilität von 111 Newton.

Beispiel 2    Herstellung von 7-ß-Phenylacetamido-3-hydroxymethyl-ceph-3-em-4-carbonsäure mit einem Bakterienpolymerisat aus Bacillus subtilis

5 g 7-ß-Phenylacetamido-3-acetoxymethyl-ceph-3-em-4-carbonsäure werden in 500 ml Wasser gelöst und bei 25°C und pH 7,0 (konstant gehalten mit 2 N $NH_3$) mit 67,5 g Bakterienpolymerisat nach Beispiel 1 in 8 h gespalten. Die Suspension wird abgesaugt, das Bakterienpolymerisat dreimal mit entsalztem Wasser gewaschen und getrocknet. Es werden 66,74 g (98,9 %) des eingesetzten Bakterienpolymerisates zurückgewonnen.

Das Filtrat wurde mit ents. Wasser auf ca. 1 l verdünnt, mit HCl auf pH 4 eingestellt und mit je 300 ml und 2 x 200 ml Essigester extrahiert.

Die organischen Phasen wurden vereinigt, mit 40 ml $H_2O$ gewaschen, über $Na_2SO_4$ getrocknet und am Rotationsverdampfer auf ca. 20 ml konzentriert. Der Rückstand wurde abgesaugt und getrocknet.

Nach dreimaligem Einsatz werden folgende Ausbeuten an 7-ß-Phenylacetamido-3-hydroxymethyl-ceph-3-em-carbonsäure erhalten:

Le A 23 204

|  |  |  | NH$_3$-Verbrauch: |
|---|---|---|---|
| 1. Spaltung | Ausbeute = | 1,55 g (43,4 %) | 3,76 ml |
| 2. Spaltung | Ausbeute = | 2,31 g (64,6 %) | 7,50 ml |
| 3. Spaltung | Ausbeute = | 2,74 g (76,6 %) | 9,88 ml |

Beispiel 3   Mehrfacher Einsatz von Bakterienpolymerisat zur Herstellung von 7-ß-Phenylacetamido-3-hydroxymethyl-ceph-3-em-4-carbonsäure

Eine 10 gew.-%ige wäßrige Lösung von 7-ß-Phenylacetamido-3-acetoxymethyl-ceph-3-em-4-carbonsäure wird bei 25°C und bei konstantem pH-Wert von 7 (Neutralisation mit 25 %iger Ammoniak-Lösung) mit 450 g, nach Beispiel 1 hergestelltem Bacillus subtilis, Bakterienpolymerisat 22 Stunden unter Rühren gespalten. Der Biokatalysator wird abfiltriert, mit Wasser gewaschen und die wäßrige Lösung nach Ansäuern auf pH 2 mit Essigester extrahiert. Nach dem Abdampfen des Essigester wird die Ausbeute an 7-ß-Phenylacetamido-3-hydroxymethyl-ceph-3-em-4-carbonsäure ausgewogen und der Biokatalysator mehrfach zur Spaltung eingesetzt.

Le A 23 204

| Einsatz des Biokatalysators | Menge 7-ß-Phenylacetamido-3-acetoxymethyl-ceph-3-em-4-carbonsäure (g) | Ausbeute 7-ß-Phenylacetamido-3-hydroxymethyl-ceph-3-em-4-carbonsäure (g) |
|---|---|---|
| 1 | 100 | 27,3 (32 % d.Th.) |
| 2 | 100 | 46,2 (55 % d.Th.) |
| 3 | 100 | 50,4 (60 % d.Th.) |
| 4 | 100 | 51,7 (61 % d.Th.) |
| 5 | 100 | 56,7 (67 % d.Th.) |
| 6 | 100 | 58,0 (69 % d.Th.) |
| 7 | 100 | 57,0 (68 % d.Th.) |
| 8 | 100 | 55,7 (66 % d.Th.) |
| 9 | 100 | 53,7 (64 % d.Th.) |
| 10 | 100 | 46,7 (55 % d.Th.) |
| 11 | 100 | 51,9 (61 % d.Th.) |
| 12 | 100 | 57,9 (68 % d.Th.) |
| 13 | 100 | 51,5 (61 % d.Th.) |
| 14 | 100 | 58,7 (69 % d.Th.) |
| 15 | 100 | 52,6 (62 % d.Th.) |
| 16 | 100 | 51,6 (61 % d.Th.) |

Beispiel 4    Herstellung von 7-β-/N̄'-tert.-Butoxycar-
              bonylamid͟o/-3-hydroxymethyl-ceph-3-em-4-
              carbonsäure

Eine 10 gew.-%ige wäßrige Lösung von 7-β-/N̄'-tert.-But-
oxycarbonylamid͟o/-3-acetoxymethyl-ceph-3-em-4-carbonsäure wird
mit 633 g nach Beispiel 1 immobilisierten Bacillus subtilis-Zellen bei 25°C und einem konstanten pH-Wert von
7,0 (konstant gehalten mit 25 %iger NH$_3$-Lösung) in 16 h
zu 7-β-/N̄'-tert.-Butoxycarbonylamid͟o/-3-hydroxymethyl-
ceph-3-em-4-carbonsäure gespalten. Diese wird, wie in
Beispiel 3 angegeben, isoliert.

Le A 23 204

Le A 23 204

| Einsatz des Biokatalysators | Menge 7-ß-/$\overline{\text{N}}$'-tert.-Butoxymethyl-carbonylamid$\underline{\text{o}}$/-3-acetoxymethyl-ceph 3-em-4-carbonsäure (g) | Menge 7-ß-/$\overline{\text{N}}$'-tert.-Butoxy-carbonylamid$\underline{\text{o}}$/-3-hydroxymethyl-ceph-3-em-4-carbonsäure (g) |
|:---:|:---:|:---:|
| 1 | 14,9 | 8,9 (67,3 % d.Th.) |
| 2 | 45,0 | 27,0 (67,5 % d.Th.) |
| 3 | 100,0 | 61,7 (69,5 % d.Th.) |
| 4 | 100,0 | 76,5 (86,2 % d.Th.) |
| 5 | 56,8 | 50,0 (99,1 % d.Th.) |

0173206

Beispiel 5    Herstellung von 7-ß-/N̄'-tert.-Butoxycarbony̲l̲7-D-(α̇)-phenylglycinamido-3-hydroxymethyl-ceph-3-em-4-carbonsäure

Eine 6 gew.-%ige wäßrige Lösung von 7-ß-/N̄'-tert.-Butoxycarbony̲l̲7-D-(α̇)-phenylglycinamido-3-acetoxymethyl-ceph-3-em-4-
carbonsäure wird mit 25 %iger NH₃-Lösung auf pH 7,0 eingestellt und mit 633 g nach Beispiel 1 immobilisierten
Zellen von Bacillus subtilis bei 25°C und einem konstanten pH-Wert von 7,0 (konstant gehalten mit 25 %iger NH₃-
Lösung) in 16 h zu 7-ß-/N̄'-tert.-Butoxycarbony̲l̲7-D-(α̇)-
phenylglycinamido-3-hydroxymethyl-ceph-3-em-4-carbonsäure gespalten. Diese wird, wie in Beispiel 3 angegeben,
isoliert.

Le A 23 204

| Einsatz des Biokatalysators | Menge 7-ß-/N̄'-tert.-Butoxy-carbonyl̲/̄-D̄-(α)-phenylglycin-amido-3̄-acetoxymethyl-ceph-3-em-4-carbonsäure | Menge 7-ß-/N̄'-tert.-Butoxy-carbonyl̄/-D̄-(α)-phenylglycin-amido-3̄-hydroxymethyl-ceph-3-em-4-carbonsäure |
|---|---|---|
| | (g) | (g) |
| 1 | 20 | 14,15 (77 % d.Th.) |
| 2 | 20 | 18,0 (98 % d.Th.) |
| 3 | 20 | 14,7 (80 % d.Th.) |
| 4 | 20 | 15,3 (83 % d.Th.) |

Die N'-tert.-Butoxycarbonylgruppe kann in bekannter Weise sauer mit Trifluoressigsäure abgespalten werden.

0173206

Beispiel 6    Herstellung von 7-ß-Amino-3-hydroxymethyl-
             ceph-3-em-4-carbonsäure mit immobilisiertert Penicillinacylase

138,7 g der nach Beispiel 3 hergestellten 7-ß-Phenyl-acetamido-3-hydroxmyethyl-ceph-3-em-4-carbonsäure werden als 3 gew.-%ige Lösung in ents. Wasser gelöst und bei 37°C und pH 7,0 in 9 Ansätzen mit jeweils 25 - 40 g feuchtem, nach DOS 2 355 078 hergestelltem Penicillinacylase-Harz gespalten und die 7-ß-Amino-3-hydroxymethyl-ceph-3-em-4-carbonsäure bei pH 4 unter Zusatz von 20 Vol.-% Isopropanol isoliert.

Ausbeute: 54,4 g.

Le A 23 204

<u>Patentansprüche</u>

1. Verfahren zur Immobilisierung von Bacillus subtilis-Zellen, dadurch gekennzeichnet, daß man eine Bacillus subtilis-Suspension zur Ausfällung und Fixierung der Zellen mit dem Fällmittel Tannin, dem Polyethylenimin und Betz 1180 sowie dem Quervernetzungsmittel Glutardialdehyd versetzt.

2. Verfahren gemäß Anspruch 1, dadurch gekennzeichnet, daß man Volumenanteile von 2 - 8 Vol.-% Tanninlösung (4 Gew.-%), 0,5 - 5 Vol.-% Polyethylenimin (4 Gew.-%) und 2,0 - 5,5 Vol.-% Betz/Glutardialdehyd (6 Gew.-% Betz, 2,2 Vol.-% Glutardialdehyd-Lösung) verwendet.

3. Verfahren gemäß Anspruch 1 und 2, dadurch gekennzeichnet, daß zur Immobilisierung direkt Fermentationsbrühen verwendet werden.

4. Immobilisierte Bacillus subtilis-Zellen erhältlich nach dem Verfahren gemäß Anspruch 2.

5. Stabile Biokatalysatoren, bestehend aus biologischem Material gemäß Anspruch 4.

6. Verwendung des biologischen Materials gemäß Anspruch 4 zur Transformation von reversibel geschützten 7-ß-Acylamido-cephalosporansäuren in 7-ß-Acylamido-3-hydroxymethyl-ceph-3-em-4-carbonsäuren.

<u>Le A 23 204</u>

Europäisches
Patentamt

# EUROPÄISCHER RECHERCHENBERICHT

## EINSCHLÄGIGE DOKUMENTE

EP 85110356.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | <u>EP - B1 - 0 053 764</u> (MILES)<br><br>* Seite 5, Zeile 50 - Seite 6, Zeile 24; Patentansprüche 1, 3-9,11,12 * | 1-5 | C 12 N 11/02<br>C 12 N 11/08<br>C 12 P 35/00<br>//C 12 R  1:125 |
| A | <u>US - A - 4 390 627</u> (LANTERO)<br><br>* Spalte 2, Zeile 17 - Spalte 4, Zeile 48 * | 1-4 | |
| D,A | <u>DE - A1 - 2 355 078</u> (BAYER)<br><br>* Anspruch 1 * | 6 | |
| A | <u>GB - A - 2 060 610</u> (GLAXO)<br><br>* Ansprüche 1,9 * | 6 | |
| A | <u>DE - A - 2 423 058</u> (GLAXO)<br><br>* Ansprüche 1,9 *<br><br>---- | 6 | RECHERCHIERTE SACHGEBIETE (Int. Cl.4)<br><br>C 12 N<br>C 12 P |

Der vorliegende Recherchenbericht wurde für alle Patentansprüche erstellt.

| Recherchenort<br>WIEN | Abschlußdatum der Recherche<br>04-12-1985 | Prüfer<br>BECKER |
|---|---|---|